# EUROPÄISCHE PATENTSCHRIFT

(11) **EP 0 618 961 B1**
(45) Veröffentlichungstag und Bekanntmachung des Hinweises auf die Patenterteilung: **23.06.1999**
(21) Anmeldenummer: 93901019.5
(22) Anmeldetag: 15.12.1992
(51) Int. Cl.: C12N 9/02

(54) **VERFAHREN ZUR ISOLIERUNG VON ENZYMEN AUS PILZEN UND ENZYMGEMISCH**
PROCESS FOR ISOLATING ENZYMES FROM MUSHROOMS AND ENZYMES MIXTURES THUS OBTAINED
PROCEDE D'ISOLEMENT D'ENZYMES CONTENUES DANS DES CHAMPIGNONS ET MELANGE D'ENZYMES AINSI OBTENU

(30) Priorität: 18.12.1991 DE 4141901
(43) Veröffentlichungstag der Anmeldung: 12.10.1994
(73) Patentinhaber: Grabbe, Klaus, Dr., D-38116 Braunschweig (DE); CORDES, Arno, D-38239 Salzgitter 1 (DE)
(72) Erfinder: Grabbe, Klaus, Dr., D-38116 Braunschweig (DE); CORDES, Arno, D-38239 Salzgitter 1 (DE)
(74) Vertreter: von Samson-Himmelstjerna, Friedrich R., Dipl.-Phys.
(86) Internationale Anmeldenummer: EP9202911
(87) Internationale Veröffentlichungsnummer: WO9312222

(56) Entgegenhaltungen:
- Patent Abstracts of Japan, Band 13, Nr 278, C-611,, Zusammenfasung von JP, 01-74984 (DAIKIN IND LTD),
- Chemical Abstracts, Band 109, Nr 17, 24 Oktober 1988, (Columbus, Ohio, US), Mokeeva V.L. et al., "Oxidases of fruit bodies of basidiomycetes", 434, Zusammenfassung 146360h, Mikol. Fitopatol. 1988, 22 17), 235-239, (e)
- Dialog Information Services, File 351, WPI, Dialog accession no. 007383522, WPI accession no. 88-017457/03, TAKARA SHUZO KK: "Prepn. of poly- phenoloxidess - by culturing negative stain of Rigidosporous zonalis and recovering the enzyme", JP 62278981, A, 871203, 8803 (Basic)
- Chemical Abstracts, Band 111, Nr 15, 9 Oktober 1989, (Columbus, Ohio, US), Kantelinen, Anne et al, "Production of lignin peroxidase and laccase by Phlebia radiata", 617, Zusammenfassung 132505p, Appl. Microbiol. Biotechnol. 1989, 31 15), 234-239, (e)

## Beschreibung

Die bisher bekannten fermentativen Verfahren zur Herstellung von Laccase (Polyphenoloxidasen) aus Pilzen leiden unter geringen Raum-Zeit-Ausbeuten sowie hohen Produktionskosten (lange Wachstumszeiten des Pilzmycels, Sterilbedingungen) (1, 2, 3, 4, 5).

Die Induzierung der Laccase wurde in zahlreichen Arbeiten (1, 2, 3, 4, 5) beschrieben. Die hier beschriebenen Induktoren sind jedoch entweder gar nicht bekannt (Tee-Extrakt) oder (wie im Fall der Lignin-Spaltstücke) nur für Polystictus versicolor beschrieben (6). Erhöhung der Enzymausbeute durch Induktoren in Pilz-Fruchtkörpern oder deren Teilen ist bisher jedoch unbekannt.

Die Inhibierung der Laccase durch Tannine in den unterschiedlichen Phasen des Reifeprozesses sowie deren Bindung durch "Tannin-Abfänger" wurde bisher nur für Bananen beschrieben (7). Die Verwendung derartiger "Abfänger" wie z.B. Casein wurde weder bei der fermentativen Laccase-Produktion noch bei der extraktiven Herstellung aus anderen Früchten beschrieben (8, 9, 10).

Die Isolierung aus Fruchtkörpern von Pilzen ist nur einmal beschrieben (11). Dieses Verfahren hat jedoch folgende Nachteile:
1. Die Fruchtkörper müssen homogenisiert werden. Daraus resultiert eine höhere Anzahl an unerwünschten Verbindungen (Chelat-Bildner, die Kupfer komplexieren; Inhibitoren; kolloidal gelöste Stoffe, die möglicherweise die Laccase-Abtrennung erschweren).
2. Die Pilz-Fruchtkörper müssen vor der Verwendung eingefroren werden.
3. Die hier benötigten organischen Lösungsmittel (ca. 8 Liter Äthanol/kg Champignon-Stiele) verteuern das Verfahren erheblich.
4. Es sind zwei Filtrationsschritte notwendig, um das Rohenzym von den Zelltrümmern abzutrennen.
5. Das durch Homogenisierung freigesetzte Rohenzym ist eindeutig instabiler in Lösung wie unten in Tab. 2 belegt wird. Da jedoch für viele Einsatzbereiche ein kostengünstiges, in Tonnen-Mengen herstellbares Rohprodukt vorteilhaft, wenn nicht gar unbedingt notwendig ist, wird die Durchsetzbarkeit des Enzyms im Markt hierdurch stark in Frage gestellt.

Ziel des neu entwickelten Verfahrens war es also, diese Nachteile zu beseitigen und die Enzymausbeuten zu erhöhen.

Dieses Ziel wird durch ein Verfahren gemäß Anspruchen 1 oder 2 erreicht wobei in den Ansprüchen 3 - 19 bevorzugte Ausführungsformen beschrieben werden. In Anspruch 20 wird ein durch die erfindungsgemäßen Verfahren erhaltenes Enzymgemisch offenbart. Das im Verfahren hat gegenüber dem unter (11) beschriebenen Verfahren folgende Vorteile:
1. Die Enzymabtrennung wird durch die Verwendung von Fruchtkörper-Teilen vereinfacht, da der Hauptteil der Zellinhaltsstoffe in den Fruchtkörpern verbleibt. Außerdem bedeutet die Freisetzung des Enzyms durch einfaches Rühren geringere Investitionskosten im technischen Maßstab.
2. Es können auch frische Fruchtkörper verwendet werden.
3. Es wird ohne organische Lösungsmittel gearbeitet.
4. Es ist nur ein Filtrations- oder Zentrifugationsschritt zur Abtrennung des Rohenzyms notwendig.
5. Die Verwendung von Induktoren erhöht die Enzymausbeute.
6. Die Verwendung von Casein als "Tannin-Abfänger" erhöht die Ausbeute.
7. Die Zugabe von Cu-Salzen erhöht die Ausbeute.
8. Die Zugabe von Hexosen, insbesondere Mannosen, erhöht die Ausbeute.

Die Zugabe von 1,7 mg/ml Vanillinsäure zum jeweils verwendeten Puffer erhöht die Ausbeute um durchschnittlich 34% (8 Versuche), die von 5 mg/ml Tee-Extrakt um 67% (2 Versuche).

Die Ausbeuten im Vergleich zum Verfahren, wie unter (11) beschrieben, sind um mehr als 100% erhöht.

In Tab. 1 sind einige Ergebnisse dargestellt, die die unterschiedlichen Aktivitäten einzelner Chargen sowie die Wirkung von Vanillinsäure und Tee-Extrakt belegen.

**Tab. 1:**

| Maximale Laccase-Aktivitäten, Einfluß von Induktoren | | |
|---|---|---|
| Chargen-Nr. ¹ | Max. Laccase-Aktivität (U/g Ch.St.) | Verfahrensbeschreibung |
| 1.2. | | |
| a) | 133 | 7 Tage eingefrorene Stiele, 0,05 M KPi-Puffer, pH 7,0, 0,05 mg/ml CuSO₄, 24 Stunden rühren |
| b) | 161 | wie 1.2. a) + 1,7 mg/ml Vanillinsäure |

| 1.3. | | |
|---|---|---|
| a) | 29 | 20 Tage eingefrorene Stiele, sonst wie 1.2. a) |
| b) | 51 | wie 1.3. a) + 1,7 mg/ml Vanillinsäure |

| 2.2. | | |
|---|---|---|
| a) | 114 | wie 1.2. a) |
| b) | 142 | wie 2.2. a) + 1,7 mg/ml Vanillinsäure |

| 3.1. | | |
|---|---|---|
| a) | 81 | frische Stiele, + Casein, sonst wie 1.2, a) |
| b) | 133 | wie 3.1.a) + Vanillinsäure |
| c) | 149 | wie 3.1.a) + Tee-Extrakt |

| 4.1. | | |
|---|---|---|
| a) | 42 | wie 3.1.a) |
| b) | 60 | wie 3.1.b) |
| c) | 63 | wie 3.1.c) |
| d) | 31 | wie 4.1.a), aber frische Champignons |
| e) | 39 | wie 4.1.b) |
| f) | 27 | frische Stiele, entsprechend Verfahren (11) |

| | | |
|---|---|---|
| ¹ Die Laccase-Aktivitäten schwanken je nach Charge. | | |

| Aktivitätsbestimmung | |
|---|---|
| Substrat | 0,1 M Brenzkatechin-Lösung in 0,05 M Kaliumphosphat-Puffer, pH 4,7. |
| Testvolumen | 2,5 ml, Enzymprobe 0,025 - 0,1 ml |
| Temperatur | 25°C |
| Wellenlänge | 415 nm |
| Küvettenschichtdicke | 1 cm |
| Reaktionszeit | 1 Min. |
| Kalkulation der Units | 1 U entspricht einer Extinktionsänderung von 1,0/Min. |

**Tab. 2:**

| Stabilität des Laccase-Rohenzyms | | | | | |
|---|---|---|---|---|---|
| Temp. (°C) | Zeit (Std.) | Stabilisator Name | Konz. | Extraktion aus | Laccase (%) |
| 20 | 10 | - | - | Stielstücken | 96 |
| 20 | 10 | - | - | Homogenat | 52 |
| 20 | 10 | Vanillinsäure | 1,7 mg/ml | Stielstücken | 100 |
| 20 | 10 | " | " | Homogenat | 83 |
| | | | | | |
| 7 | 168 | - | - | Stielstücken | 100 |
| 7 | 168 | - | - | Homogenat | 42 |
| 7 | 168 | Vanillinsäure | 1,7 mg/ml | Stielstücken | 100 |
| 7 | 168 | " | " | Homogenat | 75 |
| | | | | | |
| 7 | 504 | " | " | Stielstücken | 100 |
| 7 | 504 | " | " | Homogenat | 26 |
| Bem.: für Aktivitätswerte, die nach Ablauf des Stabilitätstestes über 100 % des Anfangswertes betrugen, wurde 100 % gesetzt. | | | | | |

### Literatur

1. Wood, D.A., J. Gen. Microbiol., 117, 327-338 (1980)
2. Haars, A., Hüttermann, A., Arch. Microbiol., 125, 233-237 (1980
3. Karhunen et al., FEBS, 267, 6-8, (1990)
4. Maier, G., Diplomarbeit, Universität Gießen, (1988)
5. Bourbonnais, R. et al., FEBS, 267, 99-102, (1990)
6. Haider, K., Grabbe, K., Zentralblatt f. Bakteriol., 205, 91-96, (1967)
7. Haard, F.N. et al., J. Food Sci., 36, 854-857, (1971)
8. Matoo et al., Plant Physiol., 44, 308 (1969)
9. Ivanova et al., Ann. Technol. Agric., 17, 33 (1968)
10. Kuhive, A.A., Chem. Abstracts, 71, 88405 (1969)
11. DAIKIN KOGYO KK, JP 87230352, (1989)

### Beispiel 1:

### Isolierung von Laccase aus frischen Champignon-Stielen

a) 20 g frischer Champignon-Stiele werden gewaschen, grob zerkleinert, so daß die Stücke in etwa 2-10 mm (Höhe, Tiefe, Breite) aufweisen. Dann wird 70 ml Kaliumphosphat-Puffer, pH 7,0, mit 0,05 mg/ml CuSO₄, 20 mg/ml Caseinpepton zugesetzt (Gesamtvolumen etwa 100 ml).
   Nach etwa 24 Std. wird der Überstand durch Zentrifugation abgetrennt. Nach Zugabe von 10% v/v wird das so erhaltene Enzympräparat bei 4°C gelagert. Ausbeute: 77 U/g Ch.-Stiele.
b) wie a) + 1,7 mg Vanillinsäure
   Ausbeute: 88 U/g
c) wie a), aber neue Charge
   Ausbeute: 81 U/g
d) wie c) + 1,7 mg/ml Vanillinsäure
   Ausbeute: 133 U/g
e) wie c) + 5 mg/ml Tee-Extrakt
   Ausbeute: 149 U/g

### Beispiel 2:

### Isolierung von Laccase aus frischen Champignon-Hüten

a) Verfahren wie unter Beispiel 1a) (ohne Induktor) Ausbeute: 31 U/g
b) wie a) + 1,7 mg/ml Vanillinsäure
   Ausbeute: 39 U/g

### Beispiel 3:

### Isolierung von Laccase aus gefrorenen Champignon-Stielen

Champignon-Stiele, die 5 Tage bei -20°C gelagert wurden, wurden behandelt, wie unter Beispiel 1 und 2 beschrieben.
a) ohne Vanillinsäure
   Ausbeute: 114 U/g
b) mit 1,7 mg/ml Vanillinsäure
   Ausbeute: 142 U/g

### Beispiel 4:

### Induktion von Laccase in homogenisierten Champignon-Stielen

20 g frische Champignon-Stiele werden mit Puffer, wie in Beispiel 1 beschrieben, versetzt und 4 mal 15 sec im Waring Blender (jeweils 30 sec Absetzpause) homogenisiert. Der Überstand wird durch Zentrifugation abgetrennt.
a) ohne Vanillinsäure
   Ausbeute: 112 U/g
b) mit 1,7 mg/ml Vanillinsäure
   Ausbeute: 132 U/g

## Patentansprüche

1. Verfahren zur Gewinnung eines Enzymgemisches (Laccase, Polyphenoloxidase), das hauptsächlich Phenole, Polyphenole und deren Derivate umwandelt, aus den Fruchtkörpern von Pilzen oder Teilen der Fruchtkörper von Pilzen, dadurch gekennzeichnet, daß diese grob zerkleinert (Scheiben mit einer Dicke > 2 mm, Durchmesser > 2 mm) in Kaliumphosphat-Puffer (max. 1 M, pH 3 - 9) gerührt werden, der Enzymaustritt gemessen wird und nach Erreichen der maximalen Enzymaktivität der Überstand als einsatzfähiges Enzymprodukt abgetrennt und gelagert wird.

2. Verfahren zur Gewinnung eines Enzymgemisches (Laccase, Polyphenoloxidase), das hauptsächlich Phenole, Polyphenole und deren Derivate umwandelt, aus dem Substrat-Myzelgemenge oder Submersmyzel von Pilzen oder Teilen der Substrat-Myzelgemenge oder Submersmyzele von Pilzen, dadurch gekennzeichnet, daß diese grob zerkleinert (Scheiben mit einer Dicke > 2 mm, Durchmesser > 2 mm) in Kaliumphosphat-Puffer (max. 1 M, pH 3 - 9) gerührt werden, der Enzymaustritt gemessen wird und nach Erreichen der maximalen Enzymaktivität der Überstand als einsatzfähiges Enzymprodukt abgetrennt und gelagert wird.

3. Verfahren gemäß Anspruch 1 oder 2, dadurch gekennzeichnet, daß dem Kaliumphosphat-Puffer ein Induktor, der die Enzymausbeute erhöht, zugegeben wird.

4. Verfahren gemäß Anspruch 1, 2 oder 3, dadurch gekennzeichnet, daß der Induktor Lignin oder ein phenolisches Spaltstück des Lignins, wie z.B. Brauns Lignin, Protocatechusäure, Kaffeesäure, Ferulasäure ist.

5. Verfahren gemäß Anspruch 1, 2 oder 3, dadurch gekennzeichnet, daß der Induktor Vanillinsäure ist.

6. Verfahren gemäß Anspruch 1, 2 oder 3, dadurch gekennzeichnet, daß als Induktor ein Phenol- enthaltende Lebensmittel oder Abfallprodukte, z.B. Schwarztee-Extrakt eingesetzt wird.

7. Verfahren gemäß einem der Ansprüche 1 bis 6, dadurch gekennzeichnet, daß dem Puffer eine Tanninbindende Substanz, z.B. Casein oder Caseinpepton, zugesetzt wird.

8. Verfahren gemäß einem der Ansprüche 1 bis 7, dadurch gekennzeichnet, daß dem Puffer Kupfersalze wie z.B. CUSO₄ zur Abdeckung des Kupferbedarfs des Enzyms zugesetzt werden.

9. Verfahren gemäß einem der Ansprüche 1 bis 8, dadurch gekennzeichnet, daß die Fruchtkörper oder deren Teile auch im eingefrorenen Zustand verwendet werden.

10. Verfahren gemäß einem der Ansprüche 1 bis 9, dadurch gekennzeichnet, daß ein Speisepilz, z.B. Agaricus bisporus (Zuchtchampignon), als Enzymquelle verwendet wird.

11. Verfahren gemäß einem der Ansprüche 1 bis 10, dadurch gekennzeichnet, daß nur bestimmte Teile des Fruchtkörpers, z.B. Stiele, verwendet werden.

12. Verfahren gemäß einem der Ansprüche 1 bis 11, dadurch gekennzeichnet, daß zur Verbesserung der Enzymgewinnung und -ausbeute Enzympräparate, z.B. Chitinase, zugesetzt werden.

13. Verfahren gemäß einem der Ansprüche 1 bis 12, dadurch gekennzeichnet, daß dem so erhaltenen Enzymprodukt zur Verbesserung der Lagerfähigkeit ein Konservierungsmittel, z.B. Ascorbinsäure, Na-methyl-4-hydroxy-benzoat, Alkohole, Polyalkohole, Kaliumsorbat odgl. zugesetzt werden.

14. Verfahren gemäß einem der Ansprüche 1 bis 13, dadurch gekennzeichnet, daß als Konservierungsmittel Äthanol verwendet wird.

15. Verfahren gemäß einem der Ansprüche 1 bis 4 und 6 bis 14, dadurch gekennzeichnet, daß als zusätzlicher Stabilisator Vanillinsäure verwendet wird.

16. Verfahren gemäß einem der Ansprüche 1, 3, 4 und 13 bis 15, dadurch gekennzeichnet, daß eine Haltbarmachung durch Sprühtrocknung erfolgt.

17. Verfahren gemäß einem der Ansprüche 1, 3, 4 und 13 bis 15, dadurch gekennzeichnet, daß das Enzym an eine Trägermatrix fixiert wird.

18. Verfahren gemäß einem der Ansprüche 1, 3, 4 und 13 bis 15, dadurch gekennzeichnet, daß das Enzym unter Sauerstoffausschluß, z.B. in einer Stickstoffatmosphäre, gewonnen oder aufbewahrt wird.

19. Verfahren nach einem der vorstehenden Ansprüche, gekennzeichnet durch die Zugabe von Hexose, insbesondere Mannose.

20. Enzymgemisch erhältlich durch ein Verfahren nach einem der vorstehenden Ansprüche

## Claims

1. Process for recovering an enzyme mixture (laccase, polyphenoloxidase), which mainly transforms phenols, polyphenols and their derivatives, from the fruit bodies of fungi or parts of fruit bodies of fungi, characterised in that the latter are coarsely comminuted (slices having a thickness > 2 mm, diameter > 2 mm) and agitated in potassium phosphate buffer (maximum 1 M, pH 3 - 9), the enzyme emission is measured and after reaching maximum enzyme activity the supernatant is separated off and stored as a useful enzyme product.

2. Process for recovering an enzyme mixture (laccase, polyphenoloxidase), which mainly transforms phenols, polyphenols and their derivatives, from the substrate mycelium mixture or submerged mycelium of fungi or parts of the substrate mycelium mixture or submerged mycelium of fungi, characterised in that the latter are coarsely comminuted (slices having a thickness > 2 mm, diameter > 2 mm) and agitated in potassium phosphate buffer (maximum 1 M, pH 3 - 9), the enzyme emission is measured and after reaching maximum enzyme activity the supernatant is separated off and stored as a useful enzyme product.

3. Process according to claim 1 or 2, characterised in that an inductor which increases the enzyme yield is added to the potassium phosphate buffer.

4. Process according to claim 1, 2 or 3, characterised in that the inductor is lignin or a phenolic fragment of lignin, such as for example Braun's lignin, protocatechuic acid, caffeic acid, ferulaic acid.

5. Process according to claim 1, 2 or 3, characterised in that the inductor is vanillic acid.

6. Process according to claim 1, 2 or 3, characterised in that a phenol-containing foodstuff or waste products, for example black tea extract, is used as the inductor.

7. Process according to one of claims 1 to 6, characterised in that a tannin-binding substance, for example casein or casein peptone, is added to the buffer.

8. Process according to one of claims 1 to 7, characterised in that copper salts, such as for example CuSO₄, are added to the buffer to cover the copper requirement of the enzyme.

9. Process according to one of claims 1 to 8, characterised in that the fruit bodies or their parts are also used in the frozen state.

10. Process according to one of claims 1 to 9, characterised in that an edible fungus, for example Agaricus bisporus (cultivated mushroom), is used as the enzyme source.

11. Process according to one of claims 1 to 10, characterised in that only certain parts of the fruit body, for example stalks, are used.

12. Process according to one of claims 1 to 11, characterised in that enzyme preparations, for example chitinase, are added to improve enzyme recovery and yield.

13. Process according to one of claims 1 to 12, characterised in that a preservative, for example ascorbic acid, Na-methyl-4-hydroxy-benzoate, alcohols, polyalcohols, potassium sorbate or the like, are added to the enzyme product thus obtained to improve the shelf life.

14. Process according to one of claims 1 to 13, characterised in that ethanol is used as the preservative.

15. Process according to one of claims 1 to 4 and 5 to 14, characterised in that vanillic acid is used as additional stabiliser.

16. Process according to one of claims 1, 3, 4 and 13 to 15, characterised in that preservation is carried out by means of spray-drying.

17. Process according to one of claims 1, 3, 4 and 13 to 15, characterised in that the enzyme is fixed to a matrix.

18. Process according to one of claims 1 3, 4 and 13 to 15, characterised in that the enzyme is recovered or stored with exclusion of oxygen, for example in a nitrogen atmosphere.

19. Process according to one of the preceding claims, characterised by the addition of hexose, in particular mannose.

20. Enzyme mixture which can be obtained by a process according to one of the preceding claim.

## Revendications

1. Procédé pour préparer un mélange d'enzymes (laccase, polyphénoloxydase), qui transforme principalement les phénols, les polyphénols et leurs dérivés, à partir des corpuscules reproducteurs de champignons ou de parties des corpuscules reproducteurs de champignons, caractérisé en ce que, après broyage grossier (disques d'épaisseur > 2 mm et de diamètre > 2 mm), on agite ces derniers dans un tampon phosphate de potassium (max. 1 M, pH 3-9), on mesure la production d'enzymes et, quand on a atteint l'activité enzymatique maximale, on sépare le surnageant sous forme d'un produit enzymatique prêt à l'emploi, et on le stocke.

2. Procédé pour préparer un mélange d'enzymes (laccase, polyphénoloxydase), qui transforme principalement les phénols, les polyphénols et leurs dérivés, à partir du mélange substrat-mycélium ou du mycélium de submersion de champignons, ou de parties du mélange substrat-mycélium ou du mycélium de submersion de champignons, caractérisé en ce que, après broyage grossier (disques d'épaisseur > 2 mm et de diamètre > 2 mm), on agite ces derniers dans un tampon phosphate de potassium (max. 1 M, pH 3-9), on mesure la production d'enzymes et, quand on a atteint l'activité enzymatique maximale, on sépare le surnageant sous forme d'un produit enzymatique prét à l'emploi, et on le stocke.

3. Procédé selon la revendication 1 ou 2, caractérisé en ce qu'on ajoute au phosphate de potassium un inducteur qui augmente le rendement en enzymes.

4. Procédé selon la revendication 1, 2 ou 3, caractérisé en ce que l'inducteur est la lignine ou un produit de dissociation phénolique de la lignine, tel par exemple que la lignine de Braun, l'acide protocatéchique, l'acide caféique, l'acide férulique.

5. Procédé selon la revendication 1, 2 ou 3, caractérisé en ce que l'inducteur est l'acide vanillique.

6. Procédé selon la revendication 1, 2 ou 3, caractérisé en ce qu'on utilise en tant qu'inducteur des produits alimentaires ou des déchets contenant du phénol, par exemple de l'extrait de thé noir.

7. Procédé selon l'une des revendications 1 à 6, caractérisé en ce qu'on ajoute au tampon une substance formant un tanin, par exemple la caséine ou la caséine-peptone.

8. Procédé selon l'une des revendications 1 à 7, caractérisé en ce qu'on ajoute au tampon des sels de cuivre, tels par exemple que CuSO₄, pour couvrir les besoins en cuivre de l'enzyme.

9. Procédé selon l'une des revendications 1 à 8, caractérisé en ce que les corpuscules reproducteurs ou leurs parties sont utilisés même à l'état congelé.

10. Procédé selon l'une des revendications 1 à 9, caractérisé en ce qu'on utilise en tant que source d'enzyme un champignon comestible, par exemple Agaricus bisporus (champignon de Paris).

11. Procédé selon l'une des revendications 1 à 10, caractérisé en ce qu'on n'utilise que certaines parties du corpuscule reproducteur, par exemple les tiges.

12. Procédé selon l'une des revendications 1 à 11, caractérisé en ce que, pour améliorer la production des enzymes et le rendement en enzymes, on ajoute des préparations enzymatiques, par exemple de la chitinase.

13. Procédé selon l'une des revendications 1 à 12, caractérisé en ce qu'on ajoute au produit enzymatique ainsi obtenu, pour améliorer son aptitude au stockage, un conservateur, par exemple l'acide ascorbique, le méthyl-4-hydroxybenzoate de Na, des alcools, des polyalcools, du sorbate de potassium ou analogues.

14. Procédé selon l'une des revendications 1 à 13, caractérisé en ce que le conservateur utilisé est l'éthanol.

15. Procédé selon l'une des revendications 1 à 4 et 6 à 14, caractérisé en ce qu'on utilise de l'acide vanillique en tant que stabilisant supplémentaire.

16. Procédé selon l'une des revendications 1, 3, 4 et 13 à 15, caractérisé en ce qu'on procède à une stabilisation par séchage par atomisation.

17. Procédé selon l'une des revendications 1, 3, 4 et 13 à 15, caractérisé en ce que l'enzyme est fixée à une matrice support.

18. Procédé selon l'une des revendications 1, 3, 4 et 13 à 15, caractérisé en ce que l'enzyme est préparée ou conservée à l'abri de l'oxygène par exemple dans une atmosphère d'azote.

19. Procédé selon l'une des revendications précédentes, caractérisé par l'addition d'hexose, en particulier de mannose.

20. Mélange d'enzymes pouvant être obtenu par un procédé selon l'une des revendications précédentes.
